(19) Europäisches Patentamt
European Patent Office
Office européen des brevets

(11) Veröffentlichungsnummer: **0 158 246**
**B1**

# (12) EUROPÄISCHE PATENTSCHRIFT

(45) Veröffentlichungstag der Patentschrift: **11.05.88**

(51) Int. Cl.⁴: **C 07 C 45/50**

(21) Anmeldenummer: **85103833.1**

(22) Anmeldetag: **29.03.85**

## (54) Verfahren zur Herstellung von Aldehyden.

(30) Priorität: **10.04.84 DE 3413427**

(43) Veröffentlichungstag der Anmeldung: **16.10.85 Patentblatt 85/42**

(45) Bekanntmachung des Hinweises auf die Patenterteilung: **11.05.88 Patentblatt 88/19**

(84) Benannte Vertragsstaaten: **AT BE DE FR GB IT NL SE**

(56) Entgegenhaltungen:
**EP - A - 0 096 905**
**DE - A - 2 627 354**
**GB - A - 2 114 971**

(73) Patentinhaber: **Ruhrchemie Aktiengesellschaft, Bruchstrasse 219, D-4200 Oberhausen 11 (DE)**

(72) Erfinder: **Cornils, Boy, Dr. Dipl.-Chem., Friedrich-Ebert-Strasse 45, D-4220 Dinslaken (DE)**
Erfinder: **Konkol, Werner, Dr. Dipl.-Chem., Lützowstrasse 40, D-4200 Oberhausen 11 (DE)**
Erfinder: **Bach, Hanswilhelm, Dr. Dipl.-Chem., Alleestrasse 56, D-4100 Duisburg 11 (DE)**
Erfinder: **Dämbkes, Georg, Dr. Dipl.-Chem., Nibelungenstrasse 65, D-4220 Dinslaken (DE)**
Erfinder: **Gick, Wilhelm, Dr. Dipl.-Chem., Im Buschhuck 8, D-4100 Duisburg 74 (DE)**
Erfinder: **Greb, Wolfgang, Dr. Dr.-Ing., Südstrasse 181, D-4220 Dinslaken (DE)**
Erfinder: **Wiebus, Ernst, Ferdinandstrasse 77, D-4200 Oberhausen 11 (DE)**
Erfinder: **Bahrmann, Helmut, Dr. Dipl.-Chem., Rohrstrasse 48, D-4236 Hamminkeln/Brünen (DE)**

(74) Vertreter: **Reichelt, Karl-Heinz, Dr., Ruhrchemie Aktiengesellschaft Abt. PLD Postfach 13 01 60, D-4200 Oberhausen 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 158 246 B1

ACTORUM AG

**Beschreibung**

Die vorliegende Erfindung betrifft ein Verfahren zur Herstellung von Aldehyden durch Hydroformylierung von Olefinen in Gegenwart wasserlöslicher Rhodium-Komplexkatalysatoren.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher Metalle der 8. Gruppe des Periodensystems, katalysiert. Neben Kobalt, das als Katalysatormetall in grossem Umfang technische Anwendung findet, gewinnt in letzter Zeit Rhodium zunehmende Bedeutung. Im Gegensatz zu Kobalt gestattet Rhodium, die Reaktion bei niedrigem Druck auszuführen; darüber hinaus werden vorzugsweise geradkettige n-Aldehyde und nur in untergeordnetem Masse iso-Aldehyde gebildet. Schliesslich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzlich und gegebenenfalls überschüssige Liganden enthalten. Besonders bewährt haben sich als Liganden tertiäre Phosphine oder Phosphite. Ihre Anwendung ermöglicht, den Reaktionsdruck auf Werte unter 300 bar ($30 \cdot 10^3$ kPa) zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefinen mit bis zu etwa 5 Kohlenstoffatomen im Molekül, beschritten werden. Ausserdem hat sich gezeigt, dass die thermische Belastung des Destillationsgutes auch zu erheblichen Katalysatorverlusten durch Zersetzung der Rhodiumkomplexverbindungen führt.

Die geschilderten Mängel werden durch Anwendung von Katalysatorsystemen vermieden, die in Wasser löslich sind. Derartige Katalysatoren sind z.B. in der DE-PS-2627354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wässriger und organischer Phase, d.h. ohne Destillation und damit ohne zusätzliche thermische Verfahrensschritte. Neben sulfonierten Triarylphosphinen werden als Komplexbestandteile wasserlöslicher Rhodiumkomplexverbindungen auch carboxylierte Triarylphosphine eingesetzt.

Die Reaktion des Olefins mit Kohlenmonoxid und Wasserstoff läuft in der wässrigen, den Katalysator enthaltenden Phase ab.

Nach den Angaben in der DE-PS-2627354 soll der pH-Wert der wässrigen Katalysatorlösung nicht unter 2 absinken. Allgemein wird empfohlen, den pH-Wert auf einen Wert von 2 bis 13, vorzugsweise 4 bis 10, einzustellen.

Überraschenderweise hat sich gezeigt, dass innerhalb der vorbeschriebenen pH-Werte der Umsatz der Reaktionspartner und die Selektivität der Reaktion zu n-Aldehyden als den gewünschten Reaktionsprodukten sehr unterschiedlich ist. Hoher Umsatz und hohe Selektivität, d.h. eine optimale Führung der Reaktion, erfordern die Einhaltung eines eng begrenzten pH-Bereiches.

Die Erfindung besteht somit in einem Verfahren zur Herstellung von Aldehyden durch Umsetzung von aliphatischen Olefinen mit 2 bis 12 Kohlenstoffatomen mit Kohlenmonoxid und Wasserstoff in flüssiger Phase in Gegenwart von Wasser sowie von Rhodium in metallischer Form oder als Verbindung und dem wasserlöslichen Salz eines sulfonierten oder carboxylierten Triarylphosphins. Es ist dadurch gekennzeichnet, dass die Umsetzung des Olefins mit Kohlenmonoxid und Wasserstoff bei einem pH-Wert von wässriger Phase von 5,5 bis 6,2 erfolgt.

Besonders bewährt hat es sich, bei einem pH-Wert von 5,8 bis 6,0 zu arbeiten.

Die das erfindungsgemässe Verfahren kennzeichnenden pH-Werte beziehen sich auf die wässrige Phase während der Reaktion. Sie stellt im wesentlichen eine Lösung des aus Rhodium und wasserlöslichem Triarylphosphin bestehenden Katalysators dar.

Die Bestimmung des pH-Wertes erfolgt in bekannter Weise durch Messung mittels geeigneter Elektroden.

Der erfindungsgemäss einzuhaltende pH-Wert kann bereits bei der Herstellung der wässrigen Lösung des sulfonierten oder carboxylierten Triarylphosphins eingestellt werden, weil der durch die anschliessende Zugabe von Rhodium oder Rhodiumverbindung nur unwesentlich beeinflusst wird. Mono-, di- und trisulfonierte Arylphosphine lassen sich nach einem erprobten Verfahren zum Beispiel in der Weise gewinnen, dass man Arylphosphine mit Oleum sulfoniert, das Reaktionsprodukt mit Wasser verdünnt und die sulfonierten Arylphosphine aus der wässrigen Lösung mit einem wasserunlöslichen Amin, das in einem wasserunlöslichen organischen Lösungsmittel gelöst ist, extrahiert. Aus der organischen Phase wird darauf das sulfonierte Phosphin durch Behandlung mit der wässrigen Lösung einer Base wieder in die wässrige Phase überführt. Die Einstellung des geeigneten pH-Wertes kann in diesem Fall durch Zugabe einer genau bemessenen Menge Base zur organischen Phase erfolgen.

Selbstverständlich ist es aber auch möglich, den gewünschten pH-Wert in den schwach alkalisch bis schwach sauer reagierenden Katalysatorlösungen durch Zugabe von Säuren zu erhalten. Als Säuren können zu diesem Zweck anorganische Säuren wie Phosphorsäure oder Schwefelsäure,

saure Salze mehrwertiger anorganischer Säuren wie Alkalihydrogensulfat sowie wasserlösliche organische Säuren, z. B. Ameisensäure, Essigsäure, Propionsäure, Buttersäure, Verwendung finden.

Weiterhin kann der pH-Wert mit Hilfe eines Puffergemisches aus Salzen anorganischer Sauerstoffsäuren eingestellt werden.

Entsprechend dem geforderten pH-Bereich eignen sich als Puffergemische z. B. die Systeme $Na_2HPO_4/KH_2PO_4$ oder $KH_2PO_4$/Borax.

Es hat sich gezeigt, dass ein den Bereich von pH 5,5 bis 6,2 übersteigender pH-Wert der wässrigen Lösung die Selektivität der Reaktion deutlich vermindert. Diese Selektivitätseinbusse ist auf die Begünstigung der Aldolisierung zurückzuführen. pH-Werte, die geringer als die untere Grenze des beanspruchten Bereiches sind, führen zu einer Abnahme der Katalysatoraktivität.

Nach dem erfindungsgemässen Verfahren lassen sich Olefine mit 2 bis 12 Kohlenstoffatomen hydroformylieren. Diese Olefine können linear oder verzweigt sein mit einer endständigen oder innenständigen Doppelbindung. Beispiele für solche Olefine sind: Ethylen, Propylen, 1-Buten, 2-Buten, 1-Penten, 2-Methyl-1-buten, 1-Hexen, 2-Hexen, 1-Hepten, 1-Octen, 3-Octen, 3-Ethyl-1-hexen, 1-Decen, 3-Undecen, 4,4-Dimethyl-1-nonen, 1-Dodecen. Bevorzugt werden lineare Olefine mit 2 bis 8 Kohlenstoffatomen wie Ethylen, Propylen, 1-Buten, 1-Penten, 1-Hexen, 1-Hepten und 1-Octen eingesetzt.

Als Katalysator findet Rhodium in metallischer Form oder in Form einer seiner Verbindungen zusammen mit einem wasserlöslichen Phosphin Anwendung, das der allgemeinen Formel

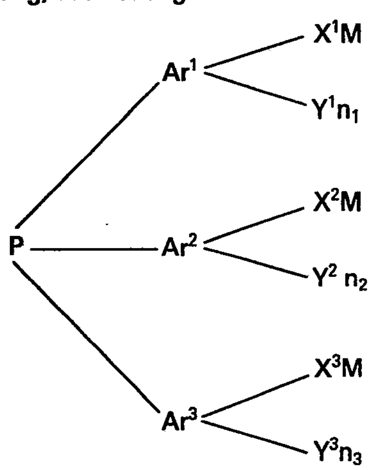

entspricht. Hierbei bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$-, oder $R^1R^2N$-Gruppen, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-($COO^-$) und/oder Sulfonat-($SO_3^-$)Rest, $n_1$, $n_2$, $n_3$ sind gleiche oder verschiedene ganze Zahlen von 0 bis 5, M ist ein Alkalimetallion, das Äquivalent eines Erdalkalimetall- oder Zinkions oder ein Ammonium- oder quaternäres Alkylammoniumion der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht.

Nach einer bevorzugten Ausführungsform dieses Verfahrens werden als wasserlösliche Phosphine Verbindungen der vorstehend beschriebenen allgemeinen Formel eingesetzt, in der $Ar^1$, $Ar^2$, $Ar^3$ jeweils einen Phenylrest und $X^1$, $X^2$, $X^3$ jeweils einen Sulfonatrest bedeuten. Beispiele für Verbindungen der oben wiedergegebenen allgemeinen Formel sind Triphenylphosphin-tri-Na-trisulfonat, Triphenylphosphin-tri-(tetraalkylammonium)-trisulfonat, Triphenylphosphin-tri-Na-tricarboxylat.

Das Rhodium wird entweder in metallischer Form oder als Verbindung eingesetzt. Metallisches Rhodium wird bevorzugt auf einem Träger wie Aktivkohle, Calciumcarbonat, Aluminiumsilikat, Tonerde aufgebracht, verwendet. Als Rhodiumverbindungen kommen solche Substanzen in Betracht, die wasserlöslich sind oder unter den Reaktionsbedingungen wasserlöslich werden. Geeignete Verbindungen sind die verschiedenen Rhodiumoxide, Salze anorganischer Wasserstoff- und Sauerstoffsäuren sowie Salze aliphatischer Mono- und Polycarbonsäuren. Als Beispiele seien genannt Rhodiumchlorid, Rhodiumnitrat, Rhodiumsulfat, Rhodiumacetat, Rhodiummalonat. Weiterhin können Rhodiumcarbonylverbindungen wie Tricarbonylrhodium oder Tetracarbonylrhodium oder Komplexsalze des Rhodiums, z. B. Cyclooctadienylrhodiumchlorid eingesetzt werden. Bevorzugt werden Rhodiumoxid, Rhodiumchlorid und Rhodiumacetat.

Die Katalysatorlösung kann im voraus, z. B. aus der wässrigen Phosphinlösung und der benötigten Menge Rhodium, hergestellt und der Reaktionszone zugeführt werden. Es ist aber ebenso möglich, die Katalysatorlösung durch Vermischen der Komponenten in der Reaktionszone selbst herzustellen.

Die Rhodiumkonzentration in der wässrigen Katalysatorlösung beträgt vorzugsweise 10 bis 2000 Gew.-ppm, bezogen auf die Lösung. Das wasserlösliche Phosphin wird in einer solchen Menge eingesetzt, dass auf 1 Grammatom Rhodium 1 bis 1000 Mol, vorzugsweise 2 bis 300 Mol, Phosphinverbindung kommen.

Der Gesamtdruck von Wasserstoff und Kohlenmonoxid beträgt 1 bis 200 bar (100 bis $20 \cdot 10^3$ kPa), vorzugsweise 10 bis 100 bar ($1 \cdot 10^3$ bis $10 \cdot 10^3$ kPa). Die Zusammensetzung des Synthesegases, d. h. das Verhältnis von Kohlenmonoxid zu Wasserstoff, kann in weiten Grenzen variiert werden. Im allgemeinen setzt man ein Synthesegas ein, in dem das Volumverhältnis von Kohlenmonoxid zu Wasserstoff 1:1 beträgt oder von diesem Wert nur wenig abweicht. Die Umsetzung erfolgt bei Temperaturen von 20 bis 150 °C, sie kann sowohl kontinuierlich als auch absatzweise durchgeführt werden.

Die folgenden Beispiele erläutern die Erfindung.

Beispiel 1

In einem kontinuierlich betriebenen 50-l-Rühr-

kessel werden bei 125 °C und einem Gesamtdruck von 50 bar ($5 \cdot 10^3$ kPa) 600 Gew.-ppm (bezogen auf die wässrige Lösung) Rhodium und so viel Propylen eingesetzt, dass entsprechend dem Olefinumsatz ein konstanter Partialdruck von Propylen, CO und $H_2$ im Verhältnis 2:1:1 eingehalten wird.

Bei Einsatz einer Katalysatorlösung mit einem pH-Wert von 5,9 können je Stunde 4,6 kg Propylen umgesetzt werden. Man erhält ein Rohprodukt folgender Zusammensetzung:

| | |
|---|---|
| i-Butanal | 3,9 Gew.-% |
| n-Butanal | 94,0 Gew.-% |
| i-Butanol | 0,1 Gew.-% |
| n-Butanol | 0,8 Gew.-% |
| 2-Ethylhexenal | 0,3 Gew.-% |
| 2-Ethylhexanal | 0,1 Gew.-% |
| $C_8$-Aldol | 0,5 Gew.-% |
| hochsiedende Verbindungen | 0,3 Gew.-% |

Beispiel 2

Unter den im Beispiel 1 angegebenen Reaktionsbedingungen wird eine Katalysatorlösung mit einem pH-Wert von 6,7 angewandt. Auch in diesem Fall lassen sich je Stunde 4,6 kg Propylen umsetzen, die Selektivität der Reaktion bezüglich der Bildung von n-Butanal verschlechtert sich jedoch ganz erheblich, wie die Zusammensetzung des Rohproduktes zeigt.

| | |
|---|---|
| i-Butanal | 3,8 Gew.-% |
| n-Butanal | 87,8 Gew.-% |
| i-Butanol | 0,1 Gew.-% |
| n-Butanol | 0,8 Gew.-% |
| 2-Ethylhexenal | 2,2 Gew.-% |
| 2-Ethylhexanal | 0,3 Gew.-% |
| $C_8$Aldol | 3,5 Gew.-% |
| hochsiedende Verbindungen | 1,5 Gew.-% |

Beispiel 3

Unter den im Beispiel 1 angegebenen Reaktionsbedingungen wird eine Katalysatorlösung mit einem pH-Wert von 5,4 angewandt. Verglichen mit Beispiel 1 wird unter diesen Bedingungen die Selektivität der Reaktion bezüglich der Bildung von n-Butanal noch verbessert. Die Aktivität geht jedoch deutlich zurück, denn je Stunde werden nur noch 3,7 kg Propylen umgesetzt. Das erhaltene Rohprodukt hat folgende Zusammensetzung:

| | |
|---|---|
| i-Butanal | 3,9 Gew.-% |
| n-Butanal | 94,6 Gew.-% |
| i-Butanol | 0,2 Gew.-% |
| n-Butanol | 1,1 Gew.-% |
| 2-Ethylhexenal | 0,1 Gew.-% |
| 2-Ethylhexanal | 0,1 Gew.-% |
| $C_8$-Aldol | 0,1 Gew.-% |
| hochsiedende Verbindungen | 0,1 Gew.-% |

**Patentansprüche**

1. Verfahren zur Herstellung von Aldehyden durch Umsetzung von aliphatischen Olefinen mit 2 bis 12 Kohlenstoffatomen mit Kohlenmonoxid und Wasserstoff in flüssiger Phase in Gegenwart von Wasser sowie von Rhodium in metallischer Form oder als Verbindung und vom wasserlöslichen Salz eines sulfonierten oder carboxylierten Triarylphosphins, dadurch gekennzeichnet, dass die Umsetzung des Olefins mit Kohlenmonoxid und Wasserstoff bei einem pH-Wert der wässrigen Phase von 5,5 bis 6,2 erfolgt.

2. Verfahren nach Anspruch 1, dadurch gekennzeichnet, dass die Umsetzung des Olefins mit Kohlenmonoxid und Wasserstoff bei einem pH-Wert der wässrigen Phase von 5,8 bis 6,0 erfolgt.

3. Verfahren nach Anspruch 1 und 2, dadurch gekennzeichnet, dass der pH-Wert durch Zusatz anorganischer Säuren, saure Salze mehrwertiger anorganischer Säuren oder durch wasserlösliche organische Säuren eingestellt wird.

4. Verfahren nach Anspruch 1 und 2, dadurch gekennzeichnet, dass der pH-Wert mit Hilfe von Puffergemischen eingestellt wird.

**Claims**

1. A process for preparing aldehydes by the reaction of aliphatic olefins having 2 to 12 carbon atoms with carbon monoxide and hydrogen in the liquid phase in the presence of water as well as rhodium in metallic form or as a compound and the water-soluble salt of a sulphonated or carboxylated triarylphosphine, characterised in that the reaction of the olefin with carbon monoxide and hydrogen takes place with the aqueous phase having a pH value of 5.5 to 6.2.

2. A process according to claim 1, characterised in that the olefin is reacted with carbon monoxide and hydrogen when the aqueous phase has a pH value of 5.8 to 6.0.

3. A process according to claims 1 and 2, characterised in that the pH value is adjusted by the addition of inorganic acids, acid salts of multivalent inorganic acids or by water-soluble organic acids.

4. A process according to claims 1 and 2, characterised in that the pH value is adjusted with the aid of buffer mixtures.

**Revendications**

1. Procédé pour la fabrication d'aldéhyde par réaction d'oléfines aliphatiques en $C_2$–$C_{12}$ avec le monoxyde de carbone et l'hydrogène en phase liquide en présence d'eau et de rhodium sous forme métallique ou sous forme de composé et du sel soluble dans l'eau d'une triarylphosphine sulfonée ou carboxylée, caractérisé en ce que la réaction de l'oléfine avec le monoxyde de carbone et l'hydrogène s'effectue à un pH de la phase aqueuse de 5,5 à 6,2.

2. Procédé selon la revendication 1, caractérisé en ce que la réaction de l'oléfine avec le monoxyde de carbone et l'hydrogène s'effectue à un pH de la phase aqueuse de 5,8 à 6,0.

3. Procédé selon la revendication 1 ou 2, caractérisé en ce que le pH est réglé par addition d'acides inorganiques, de sels acides d'acide inorganiques polyfonctionnels ou par des acides organiques solubles dans l'eau.

4. Procédé selon la revendication 1 ou 2, caractérisé en ce que le pH est réglé à l'aide de mélanges tampons.